# EUROPEAN PATENT SPECIFICATION

(11) **EP 0 534 178 B1**
(45) Date of publication and mention of the grant of the patent: **18.04.2001**
(21) Application number: 92114942.3
(22) Date of filing: 02.09.1992
(51) Int. Cl.: A61L 24/10

(54) **Improved tissue glue prepared by using cryoprecipitate**
Verbesserter Gewebekleber, zubereitet aus Kryopräzipitat
Colle améliorée pour tissus préparée à partir de cryoprécipité

(30) Priority: 27.09.1991 WO PCT/EP91/01850
(43) Date of publication of application: 31.03.1993
(73) Proprietor: OMRIX BIOPHARMACEUTICALS S.A., 1640 Rhode-St-Genése (BE)
(72) Inventor: Martinowitz, Uri, Dr., Ramat Efal 52960 (IL); Bal, Frederic, A-1080 Wien (AT)
(74) Representative: Meyers, Hans-Wilhelm, Dr.Dipl.-Chem.

(56) References cited:
- EP-A- 0 253 198
- EP-A- 0 341 007
- WO-A-86/01814
- WO-A-91/01762
- WO-A-92/15341
- DE-A- 2 201 993

## Description

This invention relates to a two component tissue glue with two separate components A and B, a process for preparing the tissue glue, the use of a high amount of aprotinin and the use of a snake venom proteolytic enzyme for preparing a tissue glue.

Improvement of local hemostasis at the site of a surgical wound by application of plasma proteins is a well-known concept. Thus, fibrin patches for hemostasis in cerebral surgery have been used. Blood plasma and thrombin were used to produce a fibrin film over the surgical wound. In the last 20 years there are a lot of publications describing applications of "fibrin glue" or "fibrin adhesive" or "fibrin sealant" for most surgical disciplines. In the last 10 years commercial preparations of "glue" are used widely in Europe. The "glue" is composed of two components, whereas a mixture of these components produce a clot. The first component is a fibrinogen concentrate. This concentrate also contains fibronectin and factor XIII which are important for clot stabilization and strength. The second component is thrombin, an active enzyme that converts fibrinogen, the last component of the normal coagulation system into a fibrin clot. This process bypasses most of the steps of normal coagulation and mimicks its last phase. Some manufacturers add plasminogen which is an enzyme that will induce clot lysis after some time whereas others add aprotinin which is an inhibitor of proteases for preventing clot lysis.

Although, these products give satisfactory results in patients although with mild bleeding disorders, but patients suffering severe bleeding disorders such as hemophilia A or B, still have a very high risk of postoperative bleeding. Sometimes a delayed bleeding complications after an average of days from the surgery occur. Alsopatients who are treated with anticoagulation factors cannot be treated with the tissue glue of the prior art. Another severe disadvantage of the commerical concentrates are the high producing costs.

WO 86/01814 discloses a method of preparing a cryoprecipitated suspension containing fibrinogen and Factor VIII useful as a precursor in the preparation of a fibrin glue which involves (a) freezing fresh frozen plasma from a single donor such as a human or other animal which has been screened for blood transmitted diseases at about -80°C for at least abount six hours; (b) raising the temperature of the frozen plasma, e.g. to between about 0°C and room temperature, so as to form a supernatant and a cryoprecipitated suspension containing fibrinogen and Facot VIII; and (c) recovering the cryoprecipitated suspension. There is also disclosed a method of preparing a fribrin glue useful in surgical procedures which comprises: (a) preparing a cryoprecipitated suspension as described above; (b) applying a defined volume of the suspension to a desired site; and (c) applying a composition containing a sufficient amount of thrombin to the site so as to cause the fibrinogen in the suspension to be converted to the fibrin glue which then solidifies.

EP-A-0 341 007 discloses a surgical adhesive comprising, in an aqueous composition, patient autogenous plasma, collagen, thrombin, and optionally, an antifibrinolytic agent. The present adhesive is formed from the patient's plasma without the use of any added reagents for concentration or isolation of the fibrinogen. Conveniently, the adhesive is formulated as a two-part composition which is mixed together just prior to use.

EP-A-0 253 198 discloses a one-component-tissue glue having an aqueous solution of fibrinogen, Factor VIII, a thrombin inhibitor, prothrombin factors, calcium ions and optionally a plasmin inhibitor. The tissue glue can be reconstituted from a lyophylized sample by adding water. The tissue glue may contain all active substances in a pateurizated from in order to avoid hepatitis and HTLV III transference.

WO-A-91/01762 discloses a process for pasteurising a mixture of proteins mainly containing fibrinogen by heating said mixture in conditions ensuring pasteurisation in which said mixture is pasteurised in the presence of monosaccharide and an alcoholic sugar. This tissue glue can be prepared from starting with cryoprecipitate. There is described a concentration step after the pasteurisation.

EP-A-0 305 243 discloses a tissue glue comprising a component A containing fibrinogen, fibrinectin, factor VIII and 10,000 KIU/ml of aprotinin and component B consisting of thrombin.

An object of the present invention is to provide a tissue glue which is also suitable for patients with severe blood coagulation disorders like hemophilia A or B. A further object of the present invention is to provide a tissue glue which can also be used for patients which have already developed antibodies against bovine thrombin which is the active factor of the component B. Still another object of the present invention is to provide a tissue glue for patients who are treated with anticoagulation factors like heparin. Because of the risk of transferring viral diseases with the components of the tissue glue it must be ensured that fractions of the tissue glue are virus inactivated.

The two component tissue glue with two separate components A and B according to the invention comprises a component A which comprises a cryoprecipitate of whole blood and an amount of a protease inhibitor corresponding to 3,000 to 5,000 KIU/ml units of aprotinin, said component A being obtainable by
- thawing a cryopaste;
- dissolving in a buffer at pH 7.0 to 7.2;
- preheating to 30 to 35EC;
- adjusting pH to 7.0 to 7.2;
- adding aluminum hydroxide under stirring;
- centrifuging and discarding the precipitate;
- adding CaCl₂;
- virus inactivation;
- concentrating by ultrafiltration to a protein concentration of 60 to 100 mg/ml;
- adding an amount of a protease inhibitor corresponding to 3,000 to 5,000 KIU/ml units of aprotinin;
and a component B, comprising a proteolytic enzyme being capable of cleaving specifically fibrinogen present in component A and causing the formation of a fibrin polymer.

Commercially available cryoprecipitate can be used for preparation of the tissue glue of the invention. According to the invention the cyroprecipitate has to be concentrated between a factor 2 and 5, preferably factor 3.

The addition of a protease inhibitor in sufficient concentration corresponding to an amount of 3,000 to 5,000 KIU/ml units of aprotinin to the cryoprecipitate makes the tissue glue of the invention suitable for use in patients with severe bleeding disorders. The preferred protease inhibitor is aprotinin, which is commercially available under the trademark Trasylol^{®} or Antagosan^{®}

The cryoprecipitate can be obtained from the patient himself by donating an autologous blood unit prior to the operation. This approach prevents the risk of transmission of viral infections by blood derivatives. However, in order to have a proper commercial product, the cryoprecipitate has to become virus-inactivated. A procedure for virus-inactivation is described in PCT/EP 91/00503. The basic principle is treatment of the cryoprecipitate with special detergents and removing the detergent lateron from the cryoprecipitate.

The second component, component B, of the tissue glue of the present invention is prepared by a solution of a proteolytic enzyme being capable of cleaving specifically fibrinogen. Usually thrombin has been used which was isolated from plasma of human beings or mamals such as bovine. This thrombin can be delivered in a lyophilized form. The reconstitution of thrombin occurs with a 40 mmol solution of calcium chloride. The preferred concentration of thrombin is 50 to 200 u/ml.

For preparing a fast tissue glue the thrombin solution of roughly 100 u/ml of calcium chloride will be prepared. For preparing a slow glue for example by filing of cavities, i. e. tooth extraction or sealing the cavity of transphenoided hypophisectomy the thrombin will be further dissolved to a concentration of 25 u/ml with the appropriate calcium chloride solution.

Another embodiment of the improved tissue glue of the invention comprises as component B a proteolytic enzyme which is isolated from snake venom. This embodiment is advantageous because also patients having developed antibodies against thrombin can be treated. Moreover, patients which are pretreated with heparin can be treated with the tissue glue according to the invention, because heparin does not influence the reaction of the snake venom enzyme. In a very preferred embodiment of the present invention there is used the snake venom enzyme batroxobin which can be isolated from the South American pit viper Bothrpos moujeni. Preferably component B contains 0.5 to 10 u/ml of the respective proteolytic enzyme of snake venom.

Chemically batroxobin is a single chain glycopeptide with a molecular weight of approximately 36,000. Defibrase^{R} causes cleavage of α 16 Arg/17 Gly bound in fibrinogen which causes the release of fibrinopeptide A and the formation of monomeric fibrin I.

When aprotinin is used in the amounts of the invention, also the tissue glues comprising purified fibrinogen, fibronectin and factor XIII can be used as component A. The risk of after-bleeding is then dramatically reduced.

When proteolytic proteases from snake venom are used for the preparation of component B, also "conventional" components A having fibrinogen, fibronectin and factor XIII can be used. The use of high amounts of aprotinin according to the invention is preferred. A very preferred embodiment is the combination of the component A of the invention derived from cryoprecipitate with or without high amounts of aprotinin and the component B of the invention having the proteolytic enzyme isolated from snake venom.

The process for preparing the fibrin glue of the invention comprises the steps of manufacturing component A comprising the steps of
- preparing a cryosolution from concentrated cryoprecipitate,
- a virus inactivation,
- the removal of virucidal agent,
- addition of the protease inhibitor and
- preparing a appropiate protease-solution of a protease as component B.

A cryopaste is prethawed over night at 4 to 10°C. The cryopaste is dissolved in a buffer containing sodium-chlorid trisodiumcitrate and glycin and having a pH of 7.0 to 7.2 and than heated to 30 to 35°C. The cryopaste should dissolve readily otherwise it is not suitable for the preparation. The dissolution can be speeded up by cutting the cryopaste in small pieces after thawing. After cooling the solution to almost room temperature and adjusting the pH to a value of 7.0 to 7.2 aluminiumhydroxid is added under stirring. The precipitate is centrifuged and discarded. Optionally a filtration step is carried out. Then calcium chloride is added up to the desired final concentration of calcium chloride.

For the virus inactivation the solution is heated up to 30°C. Than the detergents are added. Other stirring for some time the solution is transferred into a virus free container and left at slightly elevated temperatures for several hours without stirring.

The virucidal agents are removed by adding an amount of ricine oil and gently stirring for several minutes. When the oil-/water-phases have been seperated the solution is cooled to room temperature. The aqueous layer is withdrawn in a virussafe container and the oillayer is discarded. The aqueous layer is clarified by filtration. The pH must be checked to be 7.0 to 7.2. Then the protein solution is pumped through a reversed phase column at ambient temperature. After having measured the protein content (in the range of 10 to 60 mg/ml the eluate is concentrated by ultrafiltration to a protein content of 60 to 100 mg/ml and dialysed against a buffer which is identical to the buffer mentioned above but having additionally a relatively high concentration of calcium chloride. Then the protease inhibitor is added. A sterile filtration is carried out and the sample is filled and deep frozen in suitable containers.

Component B is preferably a freeze dried protease. Particularly preferred is lyophilized thrombin or lyophilized fraction of the South American pip viper Bothrpos moujeni. The proteolyic enzyme is known under the tradename Reptilase and is the enzyme batroxobin.

The proteolytic enzymes are dissolved in a calcium chloride buffer.

The application of the two components A and B is performed using a double syringe technique for example through a plastic connector. Upon mixing of the two components a clot will be formed. The application can occur via a canula or may be sprayed to a three lumen catheter. Each one of the two components is injected into a separate lumen and an air pressure source in the range of some bars (atmospheres) is connected to the third lumen in order to spray the mixture.

The tissue glue of the invention is advantageous because it can be used with patients having severe blood coagulation disorders and being still cheaper than the known tissue glues. Patients with severe hemophilia can subsequently, for example undergo tooth extractions without preventive infusions of factor VIII concentrates with a success rate of over 80%. This means only about one fifth of the patients need infusions due to post extraction bleeding. Moreover, such patients who are pretreated with heparin can be treated with the tissue glue of the invention. Another advantage is that people who raised antibodies against thrombin the second component of the tissue glue can be treated with a tissue glue according to the invention wherein thrombin is substituted by a protease from snake venom especially Defibrase^{R} which is the serine protease batroxobin isolated from the venum of the South American pit viper Bothrpos moujeni.

The invention is further disclosed in the following examples.

Human fibrinogen (grade L) was from Kabi (Stockholm), bovine thrombin from Merz-Dade. Chromogenic substrate N-a-benzoyl-DL-arginine-p-nitroanilide (BAPNA) and analytic grade reagents were from Sigma (St. Louis, MO) . Reagents and salts were diluted with 0.015 M Tris, 0.15 M NaCl, with pH 7.4. Fibrinogen was dialyzed in Tris buffer with concentration determined from Abs₂₈₀ using a conversion factor of E^{1%} 280 = 15.

Bovine thrombin was from commercial sources (Merz-Dade or Parke Davis) with activity rating by the manufacturer. Reptilase^{R}, a snake venom which only releases FPA, was from Pentapharm (Basel). The proteolytic activity of Reptilase^{R} was normalized to that of thrombin by comparing their rates of proteolysis of a non-specific chromogenic substrate BAPNA (0.25 mM) at 37°C, in Tris/saline, pH 8.0, monitored at 405 nm for 15 minutes.

On the basis of their esterolytic activity, the unit activity of the reptilase was normalized to that of thrombin.

Fibrin glue was essentially generated by a dual syringe method with pure or cryoprecipitate fibrinogen substrate in one syringe, and reptilase (20 U/ml) or thrombin with CaCl₂ (20 mM) in the other.

Clotting time (CT) was determined with a Research Model 300-R ACL Coagulation Analyzer (IL, Milan). Viscoelasticity (TEG) was determined on a 3-channel Heiliger Thromboelastograph at 37°C. Breaking strength (BS) of glues (in grams) was determined by mixing the glue components between two pieces of coarse weaved, synthetic fiber (0.5 x 1 cm), allowing the formation of gel totally interweaved between the two pieces of coarse mesh and after 2 hours at 24°C the ensemble of mesh-glue-mesh pulled apart using an Accuforce Cadet Tensionometer (AMATEK, Mansfield & Greene, USA).

Sterile cryoprecipitate (cryo) was prepared from frozen (-30°C) human plasma which was thawed at 4°C and the supernatant plasma removed. Five such units were pooled to determine protein and fibrinogen concentrations was determined by the Buiret method before and after clotting the cryoprecipitate (diluted 1 : 5) with 2 U/mL thrombin. Factor XIII was determined by measuring [³H]-putrescine incorporation into dimethylated casein after activation of the samples with 4 U/mL bovine thrombin, 10 min, 22°C.

A notable feature of the CT-fibrinogen curve is that it is biphasic for a fixed level of thrombin or reptilase (i.e. 1 U/ml, figure 1A) and reaches a minimum in the 1 - 8 mM fibrinogen range. This differs somewhat from the maximal turbidity (after 10 min) which peaks in the range 20 to 40 mM fibrinogen. A converse experiment shows the dependency of CT on either thrombin or reptilase levels. This curve shows a near linear inverse dependency of gelling rate at low enzyme levels (less than 2 U/mL), which plateaus above at higher levels.

The development of viscoelasticity of pure fibrin is somewhat slower than its turbidity. Ca(II) is a major cofactor in gel reinforcement through factor XIIIa-induced covalent interlocking of protein chains. Such gel crosslinking is a major source of mechanical strength of the gel, which plateaus after 20 min.

A note about the ability of reptilase to induce factor XIIIa activity seems appropriate.

Protein Levels of pooled cryoprecipitate:
Pooled cryo prepared from 5 units, gave the following mean values:

| | |
|---|---|
| Protein | 75 mg/mL |
| Fibrinogen | 36 mg/mL |
| Factor XIII | 4.10 U/mL |

### Coagulation rates:

The clotting time (CT) of cryo is linearly dependent on thrombin or reptilase levels. However, above 3 U/ml, increasing enzyme levels exert little effect on CT. For a fixed level of enzyme, serial dilution of cryo, gives a biphasic CT-curve equivalent to the fibrinogen-dependency noted in the pure fibrin system.

Viscoelasticity (TEG) and Breaking Strength (BS) of Cryo Glues.

The development of viscoelasticity of cryo glues was investigated with either thrombin or reptilase. This parameter takes much longer to develop than turbidity. However, cryo glues prepared with excess of CaCl₂ and either thrombin or reptilase achieve equivalent TEG values in roughly the same time frame. It seems that after the initial onset of gelation, factor XIIIa-induced cross-linking bolsters the gel fiber structure, so that the TEG values for both glues converge within 1 hour. Similarly with the final BS of both cryo glues formed with an excess of CaCl₂. Both cryo glues break at 50 to 60g. These experiments indicate that the gel fibers within the glue become reinforced by factor XIIIa-induced, covalent cross-linking.

Preparation of a cryo-solution. Commerically cryopaste is prethawed over night at 4 to 10°C. One kilo of the cryo is dissolved in two liters of buffer A (120 mM/1 NaCl, 10 mM/l trisodiumcitrate, 120 mM/l glycin and pH 7.0 to 7.2) and preheated to 30 to 35°C. The cryopaste should dissolve readily otherwise it is not suitable for the preparation. In order to speed up the dissolution, cut the cryopaste in small pieces after thawing. Then the solution is cooled to 20°C to 22°C and the pH is checked. Optionally it must be adjusted to pH 7.0 to 7.2 by adding diluted sodiumhydroxid or acidic acid. 100 ml aluminiumhydroxid is added and stirred for another 30 minutes. The precipitated is centrifuged and discarded. The supernatant is filtrated using a 1 *µ*m filter. 0.1 M/1 CaCl₂ is added to render a final concentration of Ca²⁺ of 1 mM/l. Again the pH must be checked.
Virus inactivation.

The solution is heated up to 30°C. 1% w/v TNBP and 1% w/v Triton^{®} X 100 is added. The mixture is gently stirred for 1/2 hour. The solution is than transferred into a virusfree container and left at 30°C for 3 1/2 hours without stirring. Removal of Virucidal Agents.

150 ml Ricine oil is added to the mixture prepared as described above and stirred gently for 30 minutes. While waiting for the oil/water separation (30 to 45 minutes) the solution is cooled to 20°C. The aqueous layer is withdrawn into a virus safe container whereas the oillayer is discarded. The aqueous layer is clarified by filtration on 1 *µ*m/0.45 *µ*m filter cascade. The protein solution is than pumped through a reversed phase column (C-18-Column) at a rate of 3 liter/h at ambient temperature. The throughput is monitored by UV and collected until the absorbance has returned to 50%. The fraction contains roughly 40 mg/ml as measured in a protein assay.

The eluate is concentrated by ultrafiltration to a protein content of 70 to 80 mg/ml and dialysis against sufficient amount of a buffer B (same ingredients as buffer A but additionally 1 mM/1 calcium chloride). Then 4 mio. KIU aprotinin per liter solution is added. Afterwards a sterile filtration carried out using a 0.45 *µ*m + 0.2 *µ* m cascade. The solution is than filled and deep frozen in plastic bags, optionally lyophilized.

### Preparation of a thrombin solution

Lyophilized thrombin is dissolved in a solution of 40 mM/L calcium chloride. The amount of thrombin is 100 U/ml in the glue. For a fast working glue, for example for spraying of the glue to the area of the wound, a thrombin solution of 100 U/ml in calcium chloride will be sufficient. For a slow glue, for example filling of cavities during a tooth extraction or sealing the cavity of transphenoided hypophisectomy the thrombin will be further dissolved to a final concentration of 25 U/ml by adding great amounts of CaCl₂.

The preparation of reptilase is similar to that of thrombin. However, the amount of reptilase is roughly 2 U/ml.

### Clinical case report

The patient from the age of 21, MY (a 21 year old male) suffered from severe bleeding diathesis due to acquired inhibitor against thrombin. No background disease (i.e. tumor, or autoimmune disease) could explain this problem. Laboratory test, confirmed by two outside laboratories indicated that MY had high levels of anti-thrombin IgG anitbody. In the last year he suffered repeated attacks of renal colic due to a large stone in his left kidney pelvis. Elective lithotripsy by ultrasound was planned. Based on the technique that IgG binds to protein-A affinity columns, the patient was placed on immunosuppressive therapy combined with extra-corporal immuno-adsorption. After 8 treatments, in which 60 L of the patient's plasma was processed through passage on the protein-A column, the inhibitor titer decreased by 98%. this was determined by measuring the thrombin time (TT) of normal pooled plasma, with pre- and post-affinity purified MJ plasma. Nevertheless, the TT as well as PT and APTT values were prolonged. At this time, the kidney stone moved to the urethra, causing complete blockage of the kidney accompanied by hydronephrosis. The patient received 10 more immuno-adsorption treatments (roughly 80 liter plasma) followed by intensive plasmapheresis (roughly 50 liter) and high doese immunoglobulin infusion (2 g/kg). At this point, the thrombin-inhibitor level dereased to 0.5 %. PTT was decreased to 1.2 m (47") [vs. 2.2 - 2.3 m (85 - 90") pretreatment] and the TT was 0.9 m (35") [vs. 2.3 m (90") pre-treatment and 0.7 m (27") normal control]. It was decided to remove the stone by surgery, using biological adhesive (cryo glue) made up from cryoprecipitate and high levels (200 U/mL) of thrombin. With this mix, the cryo gelled immediately upon being sprayed. However, in the patient gelling did not occur and local hemostasis was achieved by suturing. At the end of surgery the wound looked "dry". Nevertheless, six hours later, the patient was bleeding from surgical drains. Immuno-absorption of 10 liter plasma was carried out, but with no effect on bleeding which actually increased.

The patient was re-operated to find the source of bleeding.

Though no surgical bleeding was found. Diffuse bleeding was observed from the entire wound surface areas. This time, a mix of cryo and reptilase (2 U/mL; Defibrase) was sprayed onto the wound. The spray clotted immediately, the wound surface appeared turbid and bleeding stopped. The patient continued to receive daily immuno-adsorption therapy for another 5 days, with no bleeding. This demonstrates the advantage of using the snake proteasis as component B of the tissue glue of the invention.

## Claims

1. A two component tissue glue with two separate components A and B comprising a component A which comprises a cryoprecipitate of whole blood and an amount of a protease inhibitor corresponding to 3,000 to 5,000 KIU/ml units of aprotinin, said component A being obtainable by
- thawing a cryopaste;
- dissolving in a buffer at pH 7.0 to 7.2;
- preheating to 30 to 35°C;
- adjusting pH to 7.0 to 7.2;
- adding aluminum hydroxide under stirring;
- centrifuging and discarding the precipitate;
- adding CaCl₂;
- virus inactivation;
- concentrating by ultrafiltration to a protein concentration of 60 to 100 mg/ml;
- adding an amount of a protease inhibitor corresponding to 3,000 to 5,000 KIU/ml units of aprotinin;
and a component B comprising a proteolytic enzyme being capable of cleaving specifically fibrinogen present in component A and causing the formation of a fibrine polymer.

2. The tissue glue of claim 1 wherein the protease inhibitor is aprotinin in amounts of 3,000 to 5,000 KIU/ml units.

3. The tissue glue of anyone of the claims 1 and/or 2 wherein the proteolytic enzyme is thrombin derived from mammals or human beings.

4. The tissue glue according to claim 1 comprising a component A which comprises a concentrated cryoprecipitate of whole blood, and
a component B comprising a proteolytic enzyme obtainable from snake venom which enzyme is capable of cleaving specifically fibrinogen present in component A and causing the formation of a fibrine polymer.

5. The tissue glue of claim 4 wherein the snake venom enzyme is batroxobin isolated from the venom of the South American pit viper Bothrops moojeni.

6. The tissue glue of claims 4 or 5 having high an amount of a protease inhibitor, preferably aprotinin, corresponding to 3,000 to 5,000 KIU/ml units of aprotinin.

7. The tissue glue of anyone of the claims 1 to 6 wherein the cryoprecipitate is virus inactivated.

8. Process for manufacturing a fibrin glue according to claims 1 to 7 having the steps of
- thawing a cryopaste;
- dissolving in a buffer at pH 7.0 to 7.2;
- preheating to 30 to 35°C;
- adjusting pH to 7.0 to 7.2;
- adding aluminum hydroxide under stirring;
- centrifuging and discarding the precipitate;
- adding CaCl₂;
- virus inactivation;
- concentrating by ultrafiltration to a protein concentration of 60 to 100 mg/ml;
- adding an amount of a protease inhibitor corresponding to 3,000 to 5,000 KIU/ml units of aprotinin;
and preparing an appropriate solution of a suitable protease as component B as defined in claim 1 - 7.

9. The tissue glue according to claim 1 comprising as component A fibrinogen, fibronectin and factor XIII and a protease inhibitor corresponding to an aprotinin amount of from 3,000 to 5,000 KIU/ml.

10. The tissue glue of claim 9 wherein component B is a proteolytic enzyme according to claims 4 and/or 5 or thrombin.

11. Use of an amount of a protease inhibitor corresponding to 3,000 to 5,000 KIU/ml units of aprotinin in combination with concentrated cyroprecipitate of whole blood obtainable by
- thawing a cryopaste;
- dissolving in a buffer at pH 7.0 to 7.2;
- preheating to 30 to 35°C;
- adjusting pH to 7.0 to 7.2;
- adding aluminum hydroxide under stirring;
- centrifuging and discarding the precipitate;
- adding CaCl₂;
- virus inactivation;
- concentrating by ultrafiltration to a protein concentration of 60 to 100 mg/ml;
for preparing a tissue glue of any one of claims 1 - 6.

12. Use according to claim 11, wherein the protease inhibitor is aprotinin.

13. Use of concentrated cryoprecipitate for preparing a tissue glue of any one of the claims 1 to 6.

## Patentansprüche

1. Zweikomponentiger Gewebekleber mit zwei getrennten Komponenten A und B, umfassend eine Komponente A, die ein Kryopräzipitat aus Vollblut und eine Menge eines Protease-Inhibitors, die 3000 bis 5000 kIE/ml Aprotinin entspricht, umfasst, wobei die Komponente A erhältlich ist durch
- Auftauen einer Kryopaste;
- Auflösen in einem Puffer mit pH 7,0 bis 7,2;
- Vorerhitzen auf 30 bis 35°C;
- Einstellen des pH-Werts auf 7,0 bis 7,2;
- Hinzufügen von Aluminiumhydroxid unter Rühren;
- Zentrifugieren und Verwerfen des Präzipitats;
- Hinzufügen von CaCl₂;
- Virusinaktivierung;
- Konzentrieren durch Ultrazentrifugation auf eine Proteinkonzentration von 60 bis 100 mg/ml;
- Hinzufügen einer Menge eines Protease-Inhibitors, die 3000 bis 5000 kIE/ml Aprotinin entspricht;
sowie eine Komponente B, die ein proteolytisches Enzym umfasst, das in Komponente A vorhandenes Fibrinogen spezifisch zu spalten und die Bildung eines Fibrinpolymers zu bewirken vermag.

2. Gewebekleber gemäß Anspruch 1, wobei es sich bei dem Protease-Inhibitor um Aprotinin in Mengen von 3000 bis 5000 kIE/ml handelt.

3. Gewebekleber gemäß einem der Ansprüche 1 und/oder 2, wobei es sich bei dem proteolytischen Enzym um von Säugern oder vom Menschen stammendes Thrombin handelt.

4. Gewebekleber gemäß Anspruch 1, umfassend eine Komponente A, die ein konzentriertes Kryopräzipitat aus Vollblut umfasst; und
eine Komponente B, die ein aus Schlangengift erhältliches proteolytisches Enzym umfasst, wobei das Enzym in Komponente A vorhandenes Fibrinogen spezifisch zu spalten und die Bildung eines Fibrinpolymers zu bewirken vermag.

5. Gewebekleber gemäß Anspruch 4, wobei es sich bei dem Schlangengiftenzym um Batroxobin handelt, das aus dem Gift der südamerikanischen Grubenotter *Bothrops moojeni* isoliert wurde.

6. Gewebekleber gemäß Anspruch 4 oder 5, der eine große Menge eines Protease-Inhibitors, vorzugsweise Aprotinin, die 3000 bis 5000 kIE/ml Aprotinin entspricht, enthält.

7. Gewebekleber gemäß einem der Ansprüche 1 bis 6, wobei das Kryopräzipitat virusinaktiviert ist.

8. Verfahren zur Herstellung eines Gewebeklebers gemäß den Ansprüchen 1 bis 7 mit den folgenden Schritten:
- Auftauen einer Kryopaste;
- Auflösen in einem Puffer mit pH 7,0 bis 7,2;
- Vorerhitzen auf 30 bis 35 °C;
- Einstellen des pH-Werts auf 7,0 bis 7,2;
- Hinzufügen von Aluminiumhydroxid unter Rühren;
- Zentrifugieren und Verwerfen des Präzipitats;
- Hinzufügen von CaCl₂;
- Virusinaktivierung;
- Konzentrieren durch Ultrazentrifugation auf eine Proteinkonzentration von 60 bis 100 mg/ml;
- Hinzufügen einer Menge eines Protease-Inhibitors, die 3000 bis 5000 kIE/ml Aprotinin entspricht; und
- Herstellen einer geeigneten Lösung einer geeigneten Protease als Komponente B, wie sie in den Ansprüchen 1-7 definiert ist.

9. Gewebekleber gemäß Anspruch 1, der als Komponente A Fibrinogen, Fibronectin und Faktor XIII sowie einen Protease-Inhibitor, der einer Aprotininmenge von 3000 bis 5000 kIE/ml entspricht, umfasst.

10. Gewebekleber gemäß Anspruch 9, wobei es sich bei Komponente B um ein proteolytisches Enzym gemäß den Ansprüchen 4 und/oder 5 oder um Thrombin handelt.

11. Verwendung einer Menge eines Protease-Inhibitors, die 3000 bis 5000 kIE/ml Aprotinin entspricht, in Kombination mit konzentriertem Kryopräzipitat aus Vollblut, das erhältlich ist durch
- Auftauen einer Kryopaste;
- Auflösen in einem Puffer mit pH 7,0 bis 7,2;
- Vorerhitzen auf 30 bis 35°C;
- Einstellen des pH-Werts auf 7,0 bis 7,2;
- Hinzufügen von Aluminiumhydroxid unter Rühren;
- Zentrifugieren und Verwerfen des Präzipitats;
- Hinzufügen von CaCl₂;
- Virusinaktivierung;
- Konzentrieren durch Ultrazentrifugation auf eine Proteinkonzentration von 60 bis 100 mg/ml;
zur Herstellung eines Gewebeklebers gemäß einem der Ansprüche 1 bis 6.

12. Verwendung gemäß Anspruch 11, wobei es sich bei dem Protease-Inhibitor um Aprotinin handelt.

13. Verwendung von konzentriertem Kryopräzipitat zur Herstellung eines Gewebeklebers gemäß einem der Ansprüche 1 bis 6.

## Revendications

1. Une colle pour tissus à deux composants, ayant deux composants séparés A et B comprenant un composant A qui comprend un cryoprécipité de sang entier et une quantité d'un inhibiteur de protéase correspondant à 3000 à 5000 KIU/ml en unités d'aprotinine, ledit composant A pouvant être obtenu par
- décongélation d'une cryopâte ;
- dissolution dans un tampon à pH 7,0 à 7,2 ;
- préchauffage à 30-35°C ;
- ajustement du pH entre 7,0 et 7,2 ;
- addition d'hydroxyde d'aluminium sous agitation ;
- centrifugation et rejet du précipité ;
- addition de CaCl₂ ;
- inactivation des virus ;
- concentration par ultrafiltration jusqu'à une concentration en protéine de 60 à 100 mg/ml ;
- addition d'une quantité d'un inhibiteur de protéase correspondant à 3000 à 5000 KIU/ml en unités d'aprotinine ;
et un composant B comprenant une enzyme protéolytique capable de cliver spécifiquement le fibrinogène présent dans le composant A et de provoquer la formation d'un polymère de fibrine.

2. La colle pour tissus de la revendication 1, dans laquelle l'inhibiteur de protéase est l'aprotinine en des quantités de 3000 à 5000 KIU/ml.

3. La colle pour tissus de l'une quelconque des revendications 1 et/ou 2, dans laquelle l'enzyme protéolytique est une thrombine provenant de mammifères ou d'êtres humains.

4. La colle pour tissus selon la revendication 1, comprenant un composant A qui comprend un cryoprécipité concentré de sang entier, et un composant B comprenant une enzyme protéolytique pouvant être obtenue à partir de venin de serpent, cette enzyme étant capable de cliver spécifiquement le fibrinogène présent dans le composant A et de provoquer la formation d'un polymère de fibrine.

5. La colle pour tissus de la revendication 4, dans laquelle l'enzyme de venin de serpent est la batroxobine isolée du venin de trigonocéphale d'Amérique du Sud *Bothrops Moujeni*.

6. La colle pour tissus des revendications 4 ou 5 ayant une grande quantité d'un inhibiteur de protéase, de préférence l'aprotinine, correspondant à 3000 à 5000 KIU/ml en unités d'aprotinine.

7. La colle pour tissus de l'une quelconque des revendications 1 à 6, dans laquelle le cryoprécipité a été traité pour l'inactivation des virus.

8. Procédé de fabrication d'une colle fibrineuse selon les revendications 1 à 7, comportant les étapes de
- décongélation d'une cryopâte ;
- dissolution dans un tampon à pH 7,0 à 7,2 ;
- préchauffage à 30-35°C ;
- ajustement du pH entre 7,0 et 7,2 ;
- addition d'hydroxyde d'aluminium sous agitation ;
- centrifugation et rejet du précipité ;
- addition de CaCl₂ ;
- inactivation des virus ;
- concentration par ultrafiltration jusqu'à une concentration en protéine de 60 à 100 mg/ml ;
- addition d'une quantité d'un inhibiteur de protéase correspondant à 3000 à 5000 KIU/ml en unités d'aprotinine ;
et préparation d'une solution appropriée d'une protéase appropriée comme composant B tel que défini dans les revendications 1 à 7.

9. La colle pour tissus selon la revendication 1, comprenant, comme composant A, du fibrinogène, de la fibronectine et du facteur XIII et un inhibiteur de protéase correspondant à une quantité d'aprotinine de 3000 à 5000 KIU/ml.

10. La colle pour tissus de la revendication 9, dans laquelle le composant B est une enzyme protéolytique selon les revendications 4 et/ou 5 ou une thrombine.

11. Utilisation d'une quantité d'un inhibiteur de protéase correspondant à 3000 à 5000 KIU/ml en unités d'aprotinine en association avec un cryoprécipité concentré de sang entier pouvant être obtenu par
- décongélation d'une cryopâte ;
- dissolution dans un tampon à pH 7,0 à 7,2 ;
- préchauffage à 30-35°C ;
- ajustement du pH entre 7,0 et 7,2 ;
- addition d'hydroxyde d'aluminium sous agitation ;
- centrifugation et rejet du précipité ;
- addition de CaCl₂ ;
- inactivation des virus ;
- concentration par ultrafiltration jusqu'à une concentration en protéine de 60 à 100 mg/ml ;
pour préparer une colle pour tissus de l'une quelconque des revendications 1 à 6.

12. Utilisation selon la revendication 11, dans laquelle l'inhibiteur de protéase est l'aprotinine.

13. Utilisation d'un cryoprécipité concentré pour préparer une colle pour tissus de l'une quelconque des revendications 1 à 6.
